# EUROPEAN PATENT SPECIFICATION

(11) **EP 4 399 205 B1**
(45) Date of publication and mention of the grant of the patent: **25.03.2026**
(21) Application number: 22786759.5
(22) Date of filing: 09.09.2022
(51) Int. Cl.: C07D 319/12

(54) **PROCESS FOR PURIFYING MESO-LACTIDE**
VERFAHREN ZUR REINIGUNG VON MESO-LACTID
PROCÉDÉ DE PURIFICATION DE MÉSO-LACTIDE

(30) Priority: 10.09.2021 EP 21195962
(43) Date of publication of application: 17.07.2024
(73) Proprietor: Purac Biochem B.V., 4206 AC Gorinchem (NL)
(72) Inventor: DE VRIES, Johannes Jeichienus, 4206 AC Gorinchem (NL); GOBIUS DU SART, Gerrit, 4206 AC Gorinchem (NL); DE LANG, Wilko, 4206 AC Gorinchem (NL)
(74) Representative: De Vries & Metman
(86) International application number: PCT/EP2022/075141
(87) International publication number: WO 2023/036948

(56) References cited:
- RU-C1- 2 699 801
- US-A- 5 053 485
- US-A- 5 214 159
- US-A- 5 463 086
- US-A- 5 675 021
- US-A- 5 856 523

## Description

The invention pertains to a process for purifying meso-lactide. The invention also pertains to a process for producing purified meso-lactide and a purified L-lactide and/or D-lactide stream, and to a process for producing polylactide which comprises a step of purifying meso-lactide.

Lactide, the cyclic dimer of lactic acid, is well known in the art as a starting material for polylactide polymer, which is also indicated as polylactic acid, or PLA. PLA is used in medical applications, for example in biodegradable sutures, clamps, bone plates, and biologically active controlled release devices. Additionally, PLA is an attractive polymer for many technical applications, e.g., in packaging, because it is biodegradable, and can be obtained from renewable resources. There are three stereochemical forms of lactide, namely L-lactide, which consists of two L-lactic acid monomers, D-lactide, which consists of two D-lactic acid monomers, and meso-lactide, which consists of a L-lactic acid monomer and a D-lactic acid monomer. The equimolar blend of L-lactide and D-lactide is also referred to as racemic (or rac-)lactide or D,L-lactide.

The amount of different monomers influences the properties of the resulting PLA, including its crystallization kinetics and melting point. Therefore it is conventional to regulate the relative amounts of the different lactide stereoisomers in PLA. L-lactide and D-lactide are each other's enantiomers, and have the same physical properties such as melting point and boiling point, and thus also the same distillation behaviour. Meso-lactide has different physical properties and is more volatile than either L- or D-lactide.

Conventionally, lactide is manufactured from lactic acid by a process comprising the steps of polymerising lactic acid to form lactic acid oligomers, and depolymerising the lactic acid oligomers in the presence of a catalyst, to form lactide. The lactic acid can be obtained from many sources, e.g., by subjecting a hydrocarbon source to a fermentation medium to manufacture lactic acid, followed by isolating the lactic acid.

An integrated process for manufacturing PLA starting from lactic acid is described, e.g., in Henton (Natural Fibers, Biopolymers, and Biocomposites, Edited By Amar K. Mohanty, Manjusri Misra, Lawrence T. Drzal, ISBN 9780849317415, Published April 8, 2005 by CRC Press, Chapter 16, Polylactic Acid Technology; D.E. Henton, P. Gruber, J. Lunt, and J. Randall). Lactic acid obtained through fermentation is converted to a prepolymer through condensation polymerisation. The prepolymer is then converted to lactide. The lactide is subjected to a first distillation step, intended in particular to remove water and lactic acid monomer. The lactide is then subjected to a second distillation step, in which meso-lactide is separated, resulting in on the one hand a meso-lactide-enriched stream, and on the other hand, a lactide stream which is depleted in meso-lactide, and thus enriched in L- and/or D-lactide. Portions of the two streams can be combined and provided to the polymerisation step where PLA is formed through ring-opening polymerisation.

The step of separating the meso-lactide from the L- and/or D-lactide allows the tailored provision of meso-lactide to the polymerisation reactor, resulting in the desired polymer conditions as indicated above. It also allows meso-lactide and L- and/or D-lactide to be provided for separate further uses. Meso-lactide can also be recycled back to earlier in the process, e.g., to the polymerisation or depolymerisation step.

As is well known in the art, the presence of contaminants in a polymerisation process is often associated with a detrimental effect on polymer properties. For many polymers, the presence of contaminants has been shown to lead to undesirable yellowing of the polymer. The presence of contaminants may also affect further properties of the polymer. e.g., its molecular weight. For example, Auras et al. (Poly(lactic acid): Synthesis, Structures, Properties, Processing, and Applications, 2010, states: "The specifications and allowed impurity levels of lactide monomer for PLA are defined by the polymerization mechanism and the applied catalyst. PLA is commercially produced by ROP of lactides in bulk. The tin(II)-catalyzed process offers good control over molecular weight and reaction rate provided that it is performed in the absence of impurities such as water, metal ions, lactic acid, or other organic acids. Purification of crude lactides is therefore indispensable for the industrial manufacture of high molecular weight PLA (Mw>100 kg/mol). In fact, lactide is the ultimate form of lactic acid, in its dehydrated and purest form."

In the process described above, the meso-lactide stream and the L- and/or D-lactide steam depleted in meso-lactide are obtained through two sequential distillation steps. Nevertheless, it may still be appropriate to subject either or both streams to a further purification step to remove further impurities. This is in particular the case for the meso-lactide which may contain substantial amounts of volatile components resulting from the second distillation step where it is separated from D- and L-lactide.

Accordingly, there is need in the art for a process for purifying meso-lactide. It will be evident to the skilled person that for such a process to be relevant on commercial scale there will be stringent requirements on yield and efficiency. In the context of meso-lactide, an effect which should in particular be avoided is the formation of lactic acid and linear lactic acid oligomers. On the one hand, meso-lactide has a relatively high reactivity, making the risk of formation of lactic acid and linear lactic acid oligomers a real risk. On the other, the formation of these compounds not only decreases the meso-lactide yield, their presence in themselves catalyses the conversion of lactide into lactic acid and linear lactic acid oligomers, resulting in an exacerbation of the effect. Further, any meso-lactide purification process should not generate contaminants which are difficult to remove, and it should enable the production of meso-lactide of sufficient, e.g., polymer grade, purity in an efficient manner.

The present invention provides a process which solves these problems.

The invention pertains to a process for purifying meso-lactide which has a free acid content of at least 30 meq/kg comprising at least one step of
subjecting a feed comprising at least 75 wt.% meso-lactide to solvent crystallisation followed by recovery of product meso-lactide, wherein the solvent crystallisation step is carried in a solvent comprising a total of at least 80 wt.% of at least one compound selected from the group of ketones of the formula R1-C(=O)-R2, wherein R1 and R2 are independently selected from methyl, ethyl, propyl, isopropyl, n-butyl, sec-butyl, t-butyl, and isobutyl and ethers of the formula R3-O-R4, wherein R3 and R4 are independently selected from methyl, ethyl, propyl, isopropyl, n-butyl, sec-butyl, t-butyl, and isobutyl, the solvent being present in an amount of 5-50 wt.% calculated on the total of feed and solvent, the solvent crystallisation conditions being selected such that at least 20% of the meso-lactide in the feed crystallises in solid form,
wherein the solid meso lactide recovered from a final step of solvent crystallisation followed by recovery of product meso-lactide has a free acid content of at most 20 meq/kg,
in which a total of 2-10 sequential steps of solvent crystallisation followed by recovery of product meso-lactide are carried out, and wherein at least one of the solvent crystallisation steps is carried out under the reduction of the temperature of the mixture of solvent and feed to a value below 50°C.

It has been found that the combination of the various features of the present invention results in a process which yields meso-lactide in high purity and high yield in an efficient manner, with minimum formation of lactic acid and linear lactic acid oligomers.

It is noted that the possibility of subjecting meso-lactide to solvent crystallisation has been described earlier.

US 5,053,485 describes the manufacture of a polymer of meso-lactide and, if desired, other monomers. In this reference a DL lactide is crystallized, for example, in toluene and can be separated simply from the mother liquor, mainly consisting of meso-lactide and toluene. After evaporating this mother liquor a liquid remains which consists of the meso-lactide, free acids and byproducts. Crude meso-lactide can be recovered from the solvent, and recrystallised. Ethanol, cyclohexane, toluene/cyclohexane (1:1), toluene/ethanol (1:1) and methyl ethyl ketone are mentioned as solvents, with isopropyl alcohol (IPA) indicated as preferred. It is indicated that crystallisation should be allowed to proceed rapidly because if the crystallization step lasts too long, the free acid content of the final meso-lactide will be too high, while the yield will be too low. No crystallisation examples are provided in this document.

US 5,214,159 is directed to the preparation of meso-lactide comprising separation of meso-lactide from D,L-lactide. It is indicated that meso-lactide may be recrystallised for further purification. Preferred solvents are C1-C4 alcohols, preferably isopropanol, or toluene. These references do not describe the specific solvent crystallisation procedure of the present invention.

US 5,463,086 describes a process for purifying a crude lactide in which crude lactide is recrystallised using a poor solvent and a good solvent. In the crystallisation both solvents are present at the same time. The ratio between poor solvent an good solvent is in the range of 1:1 tot 5:1. Methyl-ethyl-ketone is mentioned as an example of a good solvent. This reference does not describe crystallisation of a feed containing at least 75 wt.% of meso-lactide with 5-50 wt.% of a solvent comprising at least 70 wt.% of a ketone. This reference also does not describe the free acid content of the lactide and the amount of lactide that is to crystallise.

Other processes for the purification of cyclic esters have also been described.

US 5,856,523 describes a process for the production and purification of cyclic esters in which the purification includes the introduction of an aqueous solvent into a composition containing cyclic ester and allowing two phases to form. A first phase includes cyclic esters and any organic solvent, and a second phase includes the aqueous solvent and impurities.

US 5,675,021 is directed to methods for the production, recovery and purification of cyclic esters which comprise contacting a cyclic ester composition containing at least one impurity with at least one adsorbent to remove the impurity and recovering the purified cyclic esters.

The process according to the invention finds particular application in the manufacture of purified meso-lactide and a purified L- or D-lactide stream from a process which comprises the steps of
a) reacting lactic acid to form low molecular weight poly(lactic acid),
b) depolymerising the low molecular weight poly(lactic acid) to form a crude lactide comprising meso-lactide, and L-lactide and/or D-lactide wherein either L-lactide or D-lactide is the non-predominant lactide.

Processes of this type generally generate a crude lactide comprising meso-lactide, and L-lactide and/or D-lactide wherein either L-lactide or D-lactide is the non-predominant lactide, and as explained above, the crude lactide is often separated to form a meso-lactide stream and a stream which is depleted in meso-lactide, and which will be indicated herein as a purified L- and/or D-lactide stream. The meso-lactide stream formed in this separation step can be treated by the solvent-crystallisation process described herein. This can be done directly. Preceding crystallisation is not required.

Accordingly, the present invention also pertains to a process for producing purified meso-lactide and a purified L-lactide and/or D-lactide stream comprising the steps of
a) reacting lactic acid to form low molecular weight poly(lactic acid),
b) depolymerising the low molecular weight poly(lactic acid) to form a crude lactide comprising meso-lactide, and L-lactide and/or D-lactide wherein either L-lactide or D-lactide is the non-predominant lactide,
c) separating meso-lactide from the crude lactide in one or more steps, resulting in the formation of a meso-lactide stream comprising at least 75 wt.% of meso-lactide which has a free acid content of at least 30 meq/kg and a purified L-lactide and/or D-lactide stream,
d) and subjecting the meso-lactide stream comprising at least 75 wt.% of meso-lactide which has a free acid content of at least 30 meq/kg to a purification process, wherein the purification process is a solvent crystallisation process as presented herein, and
recovering a purified meso-lactide and a purified L-lactide and/or D-lactide stream. No intermediate crystallisation steps are carried out between the separation step c) and the solvent crystallisation process carried out in step d). In particular, the meso-lactide stream comprising at least 75 wt.% of meso-lactide which has a free acid content of at least 30 meq/kg formed in step c) may be processed directly in step d), without any intermediate purification or concentration steps.

The solvent crystallisation process as described herein is also particularly attractive for incorporation into a process for manufacturing polylactide. Therefore, the present invention also pertains to a process for producing polylactide comprising the steps of
a) reacting lactic acid to form low molecular weight poly(lactic acid),
b) depolymerising the low molecular weight poly(lactic acid) to form a crude lactide comprising meso-lactide, and L-lactide and/or D-lactide wherein either L-lactide or D-lactide is the non-predominant lactide,
c) separating meso-lactide from the crude lactide in one or more steps, resulting in the formation of a meso-lactide stream comprising at least 75 wt.% of meso-lactide which has a free acid content of at least 30 meq/kg and a purified L-lactide and/or D-lactide stream,
d) subjecting the meso-lactide stream comprising at least 75 wt.% of meso-lactide which has a free acid content of at least 30 meq/kg to a purification process, wherein the purification process is a process as presented herein, and
e) subjecting at least a portion of the purified L-lactide and/or D-lactide stream, at least a portion of the purified meso-lactide, or the combination of at least a portion of the purified L-lactide and/or D-lactide stream and at least a portion of the purified meso-lactide to a polymerisation step to form polylactide. Again, no intermediate crystallisation steps are carried out between the separation step c) and the solvent crystallisation process carried out in step d). In particular, the meso-lactide stream comprising at least 75 wt.% of meso-lactide which has a free acid content of at least 30 meq/kg formed in step c) may be processed directly in step d), without any intermediate purification or concentration steps.

Further advantages of the present invention and the various embodiments thereof will become evident from the further specification.

The invention will be discussed in more detail below.

The reason for carrying out the solvent crystallisation step is the presence of contaminants, in particular acidic contaminants. Accordingly, the meso-lactide feed as it is provided to the solvent crystallisation step has a free acid content of at least 30 meq/kg. The free acid content can be determined by means of titration using for instance sodium methylate or potassium methylate in water-free methanol.

The free acid content depends on the source of the meso-lactide, and may be much higher. In fact, the process according to the invention may be particularly attractive for processing feeds with a higher free acid content, because the process according to the invention is carried out at relatively low temperatures compared to, e.g., melt crystallisation or distillation. As indicated above, the presence of free acids increases the reactivity of the feed, and the lower temperatures of the present process help to minimise side reactions occurring during the crystallisation. Therefore, in some embodiments, the meso-lactide feed has a free acid content of at least 50 meq/kg, preferably at least 80 meq/kg, in particular at least 120 meq/kg, or at least 150 meq/kg. The upper limit is not critical. A value of 700 meq/kg may be mentioned as upper limit. In general, the free acid content will be lower, e.g., at most 400 meq/kg, in particular at most 300 meq/kg, often at most 200 meq/kg.

The starting material for the process according to the invention is a feed comprising at least 75 wt.% meso-lactide. The amount of meso-lactide is calculated on the feed as it is provided to the solvent crystallisation step. It is preferred for the feed to comprise at least 80 wt.% meso-lactide, more in particular at least 90 wt.% meso-lactide, in some embodiments at least 95 wt.% meso-lactide. The amount of meso-lactide in a lactide composition may be analysed using HPLC using water/acetonitrile mixtures as eluent and UV detection, where first lactic acid and lactoyl lactic acid will elute, then meso-lactide and at even higher elution times Land D-lactide and oligomers of lactic acid.

The feed may further comprise volatile acids like acetic and pyruvic acid, succinic acid and anhydride in amounts typically well below 1 wt%. Further compounds include 2,3-butanediol, and (esters of) other hydroxyacids that may be formed during fermentation. They may be present in tens to thousands of parts per million. The process according to the invention also reduces the presence of these compounds in the meso-lactide.

In one embodiment, the feed comprising meso-lactide is provided in liquid form. It may, e.g., be derived from a condenser following a distillation step.

The meso-lactide feed is subjected to solvent crystallisation step in a solvent comprising a total of at least 70 wt.% of at least one compound selected from the group of ketones of the formula R1-C(=O)-R2, wherein R1 and R2 are independently selected from methyl, ethyl, propyl, isopropyl, n-butyl, sec-butyl, t-butyl, and isobutyl and ethers of the formula R3-O-R4, wherein R3 and R4 are independently selected from methyl, ethyl, propyl, isopropyl, n-butyl, sec-butyl, t-butyl, and isobutyl.

As the use of a ketone or ether as disclosed herein is a key feature of the present invention, it is preferred for the solvent to consist for at least 90 wt.%, still more in particular at least 95 wt.%, even more in particular at least 98 wt.% of the total of at least one compound selected from the group of ketones and ethers as specified above.

It is preferred for the solvent to comprise less than 5 wt.%, in particular less than 2 wt.%, more in particular less than 1 wt.% of each of alcohols, esters, and aromatic solvents. The presence of alcohols and esters is disadvantageous because they may react with the lactide or contaminants present in the mixture. The presence of aromatic solvents is disadvantageous in view of environmental and food contact concerns associated therewith.

Where ketones are used of the formula R1-C(=O)-R2, wherein R1 and R2 are independently selected from methyl, ethyl, propyl, isopropyl, n-butyl, sec-butyl, t-butyl, and isobutyl, it is preferred for R1 to be methyl. It is further preferred for R1 to be methyl and R2 to be selected from methyl, ethyl, and isobutyl. It is particularly preferred for both R1 and R2 to be methyl, resulting in dimethylketone or 2-propanone, also known as acetone.

Where ethers are used of the formula R3-O-R4, wherein R3 and R4 are independently selected from methyl, ethyl, propyl, isopropyl, n-butyl, sec-butyl, t-butyl, and isobutyl, it is preferred for R1 to be methyl, ethyl, or isopropyl. It is particularly preferred for R3 and R4 to be independently selected from methyl, ethyl, or isopropyl. Diisopropylether may be mentioned as preferred.

The use of ketones has been found to be preferred over the use of ethers.

It has been found that the use of the specific solvents described herein, and the preferred solvents in particular, results in an efficient crystallisation process with a good separation of free acids impurities, where the resulting meso-lactide can be efficiently separated from the mother liquor. In particular it has been found that the meso-lactide crystal slurries formed using the specified solvents, in particular acetone, have attractive filtration properties. This means that, where crystals are formed in a slurry such as in suspension crystallisation, the crystals can be efficiently separated from the mother liquor using conventional filtration apparatus such as belt filters. Additionally, they can also be easily washed with additional solvent, which has been found to further reduce the free acid content of the meso-lactide.

As indicated above, the solvent comprises a total of at least 70 wt.% of at least one compound selected from the group of ketones and ethers as specified herein. While the use of mixed solvents of various compound is possible in principle, it is generally not considered necessary or preferred. Accordingly, in one embodiment, the solvent comprises a total of at least 70 wt.% of a compound selected from the group of ketones and ethers as specified herein, preferably at least 80 wt.%, more in particular at least 90 wt.%, still more in particular at least 95 wt.%, even more in particular at least 98 wt.%.

The meso-lactide is dissolved in the solvent in such an amount that the solution to be subject to solvent crystallisation has a solvent content of 5-50 wt.% calculated on the total of feed and solvent. It has been found that the solubility of meso-lactide in the selected solvent is so good that a limited amount of solvent, i.e., at most 50 wt.%, suffices to obtain the necessary dissolution of the meso-lactide in the solvent. Less solvent is considered preferred from a process efficiency point of the view. Therefore, in some embodiments at most 40 wt.% solvent is used, in particular at most 35 wt.%, in some embodiments at most 30 wt.%. The value of 5 wt.% has been found to be a minimum. Below this value the solubility will generally be insufficient to obtain adequate dissolution, crystallisation and separation behaviour. It may be preferred for the solvent content to be at least 10 wt.%.

The exact amount of solvent will depend on the nature of the solvent and the temperature of the mixture of solvent and meso-lactide. A higher temperature is accompanied by an increased solubility of meso-lactide in the solvent, resulting in less solvent being required to obtain a solution of meso-lactide in the solvent.

As will be evident to the skilled person, the presence of substantial amounts of water in the system is to be avoided, as water promotes the hydrolysis of lactide to lactic acid. Accordingly, it is preferred to use water-free solvents, and to otherwise take steps necessary to prevent the presence of water.

Once a solution of meso-lactide in the solvent has been obtained, the solution is brought to crystallisation conditions. Crystallisation may be promoted by decrease in temperature, by removal of solvent, or by the combination thereof, e.g., through solvent evaporation. In one embodiment, the temperature of the solution is reduced, to a value below 50°C, in particular below 45°C, more in particular below 40°C, still more in particular below 35°C, even more in particular below 30°C. From an industrial point of view working below 0°C may not be practical. A lower limit of 5°C, in particular 10°C may be mentioned. As will be evident to the skilled person, the temperature will influence crystal yield with a higher yield being obtained at lower temperatures. The suitable temperature will depend on the nature and amount of the solvent and on the desired crystal yield for the individual crystallisation step.

Depending on the saturation conditions of the solution, crystallisation may be promoted by the addition of seed crystals.

The solvent crystallisation step is carried out in such a way that at least 20% of the meso-lactide in the feed crystallises in solid form. The optimum amount will depend on the process and the ability to still efficiently separate the mother liquor containing the impurities from the solid meso-lactide. In general, crystallising lower amounts may lead to larger crystals with preferred filtration and washing properties and accordingly higher purities. On the other hand, higher amounts may be associated with higher overall process efficiency. In one embodiment, at least one, preferably all of the solvent crystallisation steps are carried out such that at least 25% of the meso-lactide in the feed crystallises in solid form, in particular at least 30%. Conversely, at high crystallization levels of above 80% slurries may become too viscous to be effectively stirred and pumped, crystal attrition may occur and liquid solid separation may become less effective. Accordingly, operating at most 80% may be preferred. The preferred level of crystallisation in a specific embodiment will also depend on solvent type and concentration and process configuration.

As indicated above, the process according to the invention is an efficient way to reduce the free acid content of the meso-lactide. This can be expressed through the reduction factor, which is defined as the free acid content of the starting material divided by the fee acid content of the product crystals. A reduction factor of 1 thus means that the free acid content is not reduced. A reduction factor of 2 means that the free acid content has been halved. It has been found that the process according to the invention makes it possible to achieve a reduction factor of at least 2, in many embodiments at least 3, or at least 4, and in some embodiments even higher, in a single crystallisation step. Based on the teachings in the present specification it is within the scope of the skilled person to select the nature and amount of the solvent and the crystallisation temperature in such a manner that the desired reduction factor for each crystallisation step can be achieved.

Suitable apparatus include the use of a static crystalliser, a scraped-wall crystallizer and a stirred vessel. Stirred vessel suspension crystallizations may be cooled by external wall cooling, or evaporative cooling, or both. or in a suspension crystalliser. Suitable apparatus is known to the skilled person and requires no further specification here. Suspension crystallisation in a stirred vessel is considered preferred, also in view of the good filtration properties of the meso-lactide crystals formed. The crystals can be recovered though centrifugation, but recovery through filtration is considered preferred. The use of a belt filter may be particularly attractive.

If so desired, the meso-lactide crystals recovered from the solvent may be washed with further solvent, to remove impurities adhering to the crystals. To prevent meso-lactide dissolving in the washing solvent, the washing solvent is generally relatively cold, i.e., at a temperature of at most the temperature where solvent crystallisation is carried out, generally lower.

In one embodiment, solvent crystallisation is carried out as suspension crystallisation, followed by recovery of the crystals by filtration, preferably on a belt filter, followed by washing of the crystals, preferably also on a belt filter. Another preferred method for the solid liquid separation step is centrifugal filtration, where mother liquor may be efficiently separated from the crystals.

It has been found that the solvent content of the crystals obtained in the process according to the invention may be relatively low, e.g., below 1 wt.%, in particular below 0.7 wt.%, more in particular below 0.5 wt.%. As a result of the relatively high volatility of the solvents, the solvent content in the crystals can easily be reduced further by evaporating the solvent, e.g., under sub-atmospheric pressure.

Depending of the amount of meso-lactide that crystallises in the crystallisation step, the mother liquor separated from the solid meso-lactide can still contain substantial amounts of meso-lactide. The mother liquor can be processed as desired. The mother liquor can be reheated and recycled to dissolve further meso-lactide. The mother liquor can also be subjected to a further crystallisation step to recover additional meso-lactide. The mother liquor can also be purged from the process, or hydrolysed to provide a technical grade lactic acid. How the mother liquor is handled will depend on the amount of meso-lactide present therein, but also on the amount of contaminants. Obviously, if the amount of contaminants in the mother liquor is too high, recycle thereof to the crystallisation process may lead to the addition rather than the reduction of contaminants in the system, which should be avoided. One advantage of the solvents used herein is that they are relatively easy to purify, e.g., through distillation. Therefore, in one embodiment, the process encompasses the step of subjecting used solvent to a distillation step, and providing purified solvent to a meso-lactide crystallisation step.

The meso-lactide crystals recovered from the solvent crystallisation process will have a contaminant content which is reduced as compared to the contaminant content of the meso-lactide as it entered the process. Nevertheless, depending on the contaminant content of the meso-lactide as it entered the process and the manner in which the process is carried out, the contaminant content may still be unacceptably high. Therefore it may be preferred for the meso-lactide recovered from the solvent crystallisation step to be subjected to a further step of solvent crystallisation followed by recovery of product meso-lactide, which process may be repeated as and when required. A total of 2-10 sequential steps of solvent crystallisation followed by recovery of product meso-lactide are carried out, in particular 2-6.

The preferences indicated above for the first solvent crystallisation and recovery step apply equally to the further solvent crystallisation and recovery steps. The steps may be carried out in the same of different manners. It is preferred to use the same solvent in all crystallisation steps, to allow efficient solvent recovery and recycle. The crystallisation conditions, e.g., the percentage of meso-lactide that crystallises in each step may differ.

In one embodiment, in a series of successive crystallizations, solvent recovered from a later crystallisation step is recycled to an earlier crystallisation step. This is possible because the solvent recovered from a later crystallisation step has a lower contaminant content than solvent recovered from earlier crystallisation steps.

For a high yield overall process, further lactide may be recovered from solvents discarded from the crystallisation steps. This may come at the cost of overall purity and the final process will be chosen such that a good balance between yield and quality is obtained.

The process according to the invention can be carried out as a batch process or as a continuous process.

In the process according to the invention, the solid meso lactide recovered from a final step of solvent crystallisation followed by recovery of product meso-lactide has a free acid content of at most 20 meq/kg. It is preferred for the free acid content to be at most 15 meq/kg, in particular at most 10 meq/kg. Depending on the specific use of the meso-lactide, it may be desired for the free acid content to be much lower, e.g., at most 5 meq/kg, at most 2 meq/kg, or at most 1 meq/kg. The allowable free acid content will also depend on the amount of meso-lactide that will be applied in the further application.

As indicated above, in one embodiment, the meso-lactide purification process of the present invention is incorporated into a process for producing purified meso-lactide and a purified L-lactide and/or D-lactide stream comprising the steps of
a) reacting lactic acid to form low molecular weight poly(lactic acid),
b) depolymerising the low molecular weight poly(lactic acid) to form a crude lactide comprising meso-lactide, and L-lactide and/or D-lactide wherein either L-lactide or D-lactide is the non-predominant lactide,
c) separating meso-lactide from the crude lactide, resulting in the formation of a meso-lactide stream comprising at least 75 wt.% of meso-lactide which has a free acid content of at least 30 meq/kg and a purified L-lactide and/or D-lactide stream,
d) and subjecting the meso-lactide stream comprising at least 75 wt.% of meso-lactide which has a free acid content of at least 30 meq/kg to a purification process,
wherein the purification process is a solvent crystallisation process as presented herein.

Steps a), b), and c) of this process are in themselves known in the art. They require little elucidation here. The following may be noted:
In step a) a low-molecular weight poly(lactic acid) is formed by condensation polymerisation of lactic acid. The lactic acid is often obtained from a biological process and generally has a high optical purity. Depending on the source it may contain at least 90% of L-lactic acid, in particular at least 95% L-lactic acid, more in particular at least 98 wt.%. Conversely, it may contain at least 90% of D-lactic acid, in particular at least 95% D-lactic acid, more in particular at least 98 wt.%. The condensation polymerisation may be carried out as is known in the art. It generally involves subjecting lactic acid to sub-atmospheric pressure, e.g. 50-500 mbar at elevated temperatures, e.g., 100-200°C, to induce polymerisation by removal of water. The average degree of polymerisation obtained is generally between 5 and 20.

The low-molecular weight polylactic acid obtained in step a) is then subjected to a depolymerisation step, to convert the low molecular weight polylactic acid to lactide. Depolymerisation is also known in the art. It is generally carried out in the presence of a catalyst. Metal-containing catalysts are often used, in particular catalysts based on tin, zinc, aluminium, lead, antimony, lead, calcium, and magnesium, e.g., in the form of halide salts or salts of organic acids, such as fatty acids. Tin (II) bis(2-ethyl hexanoate) is often used commercially. Typical concentration of lactide synthesis catalysts may be 20-2000 ppm. Reaction conditions include a temperature of 160 to 260°C, a pressure of 5 to 100 mbar, and a residence time of 10 minutes to 8 hours.

The product from the depolymerisation step is a crude lactide comprising meso-lactide, and L and/or D-lactide with L- or D-lactide being the predominant lactide and the other being the non-predominant lactide. Which lactide is the predominant lactide will depend on the stereochemistry of the starting lactic acid. In general, the L-lactide will be the predominant lactide. The crude lactide will also contain water and lactic acid.

As will be evident to the skilled person, the crude lactide will contain meso-lactide, the predominant lactide selected from L-lactide and D-lactide, and optionally, depending on the reaction conditions and any previous separation steps, the non-predominant lactide selected from D-lactide and L-lactide. In this specification this will be indicated as meso-lactide (on the one hand) and L-lactide and/or D-lactide (on the other hand).

In step c) meso-lactide is separated from the crude lactide in one or more steps, resulting in the formation of a meso-lactide stream comprising at least 75 wt.% of meso-lactide which has a free acid content of at least 30 meq/kg and a purified L-lactide and/or D-lactide stream. This separation can be carried out in one or more steps. In general, the crude lactide stream will first be subjected to a first distillation step, where water, lactic acid, and other lower-boiling impurities are removed from the system. The lactide stream can then be processed as desired, in one or more steps. In one embodiment a lactide fraction is subjected to a further distillation step resulting in the formation of a meso-lactide stream comprising at least 75 wt.% of meso-lactide which has a free acid content of at least 30 meq/kg and a purified L-lactide and/or D-lactide stream. The stereochemical purity of the purified L-lactide and/or D-lactide stream will generally be such that it consists for at least 90%, in particular at least 94%, more in particular at least 96%, still more in particular at least 98% of the dominant lactide, in particular L-lactide, calculated on the total of D- and L-lactide.

Both distillation steps, and alternative separation steps, are known in the art are require no further elucidation.

The process of this embodiment thus generates a purified meso-lactide and a purified Land/or or D-lactide stream. Both products can be processed as desired. They can, e.g., be provided to polymerisation processes for the manufacture of polymers containing lactide monomers. They can also be used for production of high purity lactic acid, as well as to be applied in coating, sealants, adhesives, resins and hot melts or production of esters.

As indicated above, in one embodiment, the meso-lactide purification process of the present invention may also be incorporated into a process for manufacturing polylactide. Therefore, the present invention also pertains to a process for producing polylactide comprising the steps of
a) reacting lactic acid to form low molecular weight poly(lactic acid),
b) depolymerising the low molecular weight poly(lactic acid) to form a crude lactide comprising meso-lactide, and L-lactide and/or D-lactide wherein either L-lactide or D-lactide is the non-predominant lactide,
c) separating meso-lactide from the crude lactide in one or more steps, resulting in the formation of a meso-lactide stream comprising at least 75 wt.% of meso-lactide which has a free acid content of at least 30 meq/kg and a purified L-lactide and/or D-lactide stream,
d) subjecting the meso-lactide stream comprising at least 75 wt.% of meso-lactide which has a free acid content of at least 30 meq/kg to a purification process, wherein the purification process is a process as presented herein, and
e) subjecting at least a portion of the purified L-lactide and/or D-lactide stream, at least a portion of the purified meso-lactide, or the combination of at least a portion of the purified L-lactide and/or D-lactide stream and at least a portion of the purified meso-lactide to a polymerisation step to form polylactide.

For steps a), b), and c) of this process, reference is made to what is stated above.

With respect to polymerisation step e), the manufacture of PLA from lactide monomers is known in the art and requires no further elucidation here. The following may be noted.

In general, polymerisation will be carried out by providing the monomers to a polymerisation reactor where they will be brought to polymerisation conditions, generally in the presence of a polymerisation catalyst. Suitable polymerisation conditions include a temperature of 100 to 225°C, in particular 130°C to 220°C, more in particular 170°C to 210°C.

Suitable catalysts are known in the art. The catalysts described above for the depolymerisation process may also be applied here. Catalysts are used in catalytically effective amounts, e.g., 1-2000 ppm, calculated on the weight of the monomer. Polymerisation will be carried out until the desired molecular weight is reached. When the desired molecular weight has been reached, the catalyst is often deactivated though the addition of a catalyst killer, to prevent the formation of free acid.

In step e) at least a portion of the purified L-lactide and/or D-lactide stream, at least a portion of the purified meso-lactide, or the combination of at least a portion of the purified L-lactide and/or D-lactide stream and at least a portion of the purified meso-lactide are subjected to a polymerisation step to form polylactide. The claim thus encompassed polymerisation of meso-lactide only, polymerisation of purified L-lactide and/or D-lactide stream only, and polymerisation of the combination of meso-lactide and purified L-lactide and/or D-lactide stream. In the latter case, the amount of meso-lactide will generally be in the range of 2-30 wt.%, calculated in the total of meso-lactide, D-lactide, and L-lactide, depending on the desired properties of the final polymer. Selection of an appropriate amount of meso-lactide is within the scope of the skilled person. Depending on the amount of meso-lactide that is available, it may also be considered to provide a portion of the meso-lactide to polymerisation step a), or depolymerisation step b). In these steps, the meso-lactide will be racemised to L-lactide and D-lactide.

The amount of meso-lactide will generally be in the range of 1-30 wt.%, calculated in the total of meso-lactide, D-lactide, and L-lactide, depending on the properties of the final polymer. Selection of an appropriate amount of meso-lactide is within the scope of the skilled person. Depending on the amount of meso-lactide that is available, it may also be considered to provide a portion of the meso-lactide to polymerisation step a), or depolymerisation step b). In these steps, the meso-lactide will be racemised to L-lactide and D-lactide.

As will be evident to the skilled person, different embodiments of the present invention can be combined unless they are mutually exclusive. When amounts, concentrations, dimensions and other parameters are expressed in the form of a range, a preferable range, an upper limit value, a lower limit value or preferable upper and limit values, it should be understood that any ranges obtainable by combining any upper limit or preferable value with any lower limit or preferable value are also specifically disclosed, irrespective of whether the obtained ranges are clearly mentioned in the context.

The following examples will illustrate the practice of the present invention in some of the preferred embodiments. Other embodiments within the scope of the claims will be apparent to one skilled in the art.

### Example 1: proof of principle

A meso-lactide feed with a meso-lactide content of >99wt.% with a free acid content of 34 meq/kg was subjected to solvent crystallisation as follows: Solid meso-lactide particles were combined with acetone as solvent. Two experiments were carried out, with 10 wt.% and 20 wt.% acetone, respectively. In each experiment, the mixture of solvent and meso-lactide was heated to a temperature which was such that all meso-lactide was dissolved in a double-jacketed, temperature controlled vessel with bottom glass filter. Then, the temperature was slowly decreased until just above a previously assessed crystallisation point. Seed crystals were added to initiate crystallisation, and the mixture was cooled until slurries were obtained with about 50% crystal content; for the 10% acetone solution that was 31°C, for 20% acetone the mixture was further cooled to 20°C. The crystals were isolated through vacuum filtration. The results of the crystallisation are given in the following table.

| | 10% acetone | 20% acetone |
|---|---|---|
| feed meso-lactide - free acid (meq/kg) | 34 | 34 |
| product meso-lactide free acid (meq/kg) | 6.2 | 5.0 |
| Reduction factor | 5.6 | 6.9 |

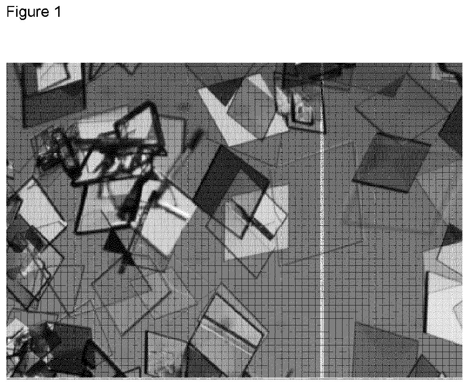

These examples show that even when starting from a relatively low free acid content the use of solvent crystallisation with acetone can yield still a substantial reduction of free acid (note that the data are on unwashed crystals). It also shows that relatively low amounts of solvent can be used. An optical microscope picture is provided as figure 1. It can be seen that platelet-shaped crystals were formed which were relatively homogeneous in size and shape. The typical crystal size was in the range of 300 microns. The filtration properties of the material were excellent, as was the removal of free acid.

### Example 2: solvent selection, crystallisation temperature, and solvent in product

In this example the starting material was a meso-lactide feed obtained from a distillation process in which meso-lactide was separated other typical components of crude lactide. The meso-lactide feed contained more than 90 wt.% meso-lactide. The free acid content varied, depending on storage conditions and times.

Crystallisation was carried out as follows: solid meso-lactide and solvent (total 1 litre) were added to a 2 litre jacketed glass filter funnel. The mixture was heated up to 55°C at which point the meso-lactide was fully dissolved in the solvent. The mixture was cooled down at 20°C/h until 2°C above the (previously determined) theoretical crystallisation temperature. The mixture was then cooled down slowly at 2°C/h. Meso-lactide seed crystals were added every 1°C. When the mixture had reached a temperature at which the seed crystals did not dissolve anymore, the mixture was kept at that temperature for 30 minutes. After 30 minutes, the mixture was further cooled at 2°C/h, until the temperature at which about 50% of the meso-lactide had crystallised (as predicted by the solubility curve), forming a slurry. Stirring was applied during the entire process.

The slurry was filtered over the glass filter and a sample was taken of the mother liquor to determine the solvent concentration. The crystals from the filter were dried in a vacuum oven.

The following table shows the crystallisation temperature and the temperature at which 50% lactide crystals are obtained, for experiments carried out at three different solvent concentrations for three different solvents. The solvent content of the final crystals is also given.

| | Crystallisation temperature (°C) | 50% crystals temperature (°C) | Crystal solvent content (wt.%) |
|---|---|---|---|
| Acetone - 5% | 45 | 35 | 0.14 |
| Acetone - 10% | 41 | 31 | 0.3 |
| Acetone - 20% | 32 | 20 | 1 |
| MIBK - 5% | 48 | 42 | 0.9 |
| MIBK - 10% | 45 | 39 | 1.6 |
| MIBK - 20% | 41 | 33 | 1.2 |
| MEK - 5% | 46 | 40 | 0.6 |
| MEK 10% | 42 | 36 | 1.2 |
| MEK - 20% | 32 | 24 | 4.2 |

From this data it can be seen that all experiments provide suitable conditions for production of slurries with high solids content, whilst giving very effective filtration operation. The solvent content of the crystals may be regarded as a measure for the filtration properties of the crystals. These values are quite low, proving good separability of crystals and mother liquor. The low amounts of solvent in the crystals furthermore provide starting points to enable a very low final amount of residual solvent after further treatment, e.g., through (vacuum) drying or distillation.

### Example 3: solvent selection and free acid content

Using the method and apparatus provided in example 2, crystallisation experiments were provided with a meso-lactide feed with a meso-lactide content of 91 wt.%. The free acid content of the feed differed per sample. The following table gives the free acid content of the feed, the free acid content of the product and the reduction factor.

| | Free acid content feed (meq/kg) | Free acid content product (meq/kg) | Reduction factor |
|---|---|---|---|
| Acetone - 5% | 44.5. | 30.8 | 1.44 |
| Acetone - 10% | 34.3 | 6.16 | 5.57 |
| Acetone - 20% | 34.3 | 4.98 | 6.89 |
| MIBK - 5% | 39.8 | 15.0 | 2.65 |
| MIBK - 10% | 32.2 | 12.8 | 2.51 |
| MIBK - 20% | 36.4 | 4.70 | 7.75 |
| MEK - 5% | 13.4 | 6.06 | 2.21 |
| MEK - 10% | 37.3 | 8.26 | 4.51 |
| MEK - 20% | 58.2 | 11.6 | 5.02 |

From this table it can be seen that even with low amounts of free acid in the starting material and low amounts of solvent a substantial reduction in the free acid content can be achieved.

### Example 4: crystal yield per pass

In this example the influence of the crystal content of the product yield was investigated. Under the conditions of Example 2, two experiments were carried out, one to obtain 50 meso-lactide crystals and one to obtain 75 % meso-lactide crystals. Acetone was used as solvent, in an amount of 10%. The following table gives the crystallisation temperature and the temperature required to obtain the desired percentage of crystals.

| | 50% crystals | 75% crystals |
|---|---|---|
| Crystallisation temperature (°C) | 39 | 39 |
| End temperature (°C) | 32 | 19 |

As expected, the temperature required to yield 75% crystals is substantially lower than the temperature required to yield 50% crystals.

### Example 5: two-step crystallisation

If the meso-lactide feed has a relatively high free acid content it may be necessary to subject the product from the first crystallisation step to a second crystallisation step. This is illustrated in this example. The feed had a meso-lactide content of more than 90%. The crystallisation was carried out using 20 wt.% acetone.

### First crystallisation

| | |
|---|---|
| Free acid content (meq/kg) | 175 |
| Crystallisation temperature (°C) | 28.5 |
| End temperature (°C) | 17 |
| Solvent content crystals (wt.%) | 2.6 |
| Free acid content product (meq/kg) | 42.5 |
| Reduction factor | 4.13 |

### Second crystallisation

| | |
|---|---|
| Free acid content (meq/kg) | 42.5 |
| Crystallisation temperature (°C) | 30 |
| End temperature (°C) | 17 |
| Solvent content crystals (wt.%) | 1.1 |
| Free acid content product (meq/kg) | 8.2 |
| Reduction factor | 5.18 |

This example shows that recrystallisation leads to a product with a decreased free acid content, and a decreased crystal solvent content. It also shows that high purification factors for free acid may be reached, irrespective of the starting amount of free acid.

### Example 6: crystal recovery through centrifugation

An experiment was carried out using 20% acetone under the conditions of Example 2, except that the crystals were separated using centrifugation rather than vacuum filtration. It appeared that centrifugation yielded crystals with a solvent content of 0.5 wt.% rather than the 1 wt.% obtained in Example 2 using the same solvent content. The free acid content of feed and product is provided in the following table.

| | |
|---|---|
| Free acid content (meq/kg) | 207 |
| Crystallisation temperature (°C) | 29 |
| End temperature (°C) | 17 |
| Solvent content crystals (wt.%) | 0.47 |
| Free acid content product (meq/kg) | 21.9 |
| Reduction factor | 9.46 |

Apparently, the low solvent content of the crystals is associated with a low free acid content of the product. This implies that the free acid is concentrated in the solvent adhering to the crystals. This means that washing the crystals with further solvent will lead to a decreased free acid content of the product.

### Example 7: static crystalliser

Solvent crystallisation was carried out under static conditions in a 1L Erlenmeyer flask. First, 500g meso-lactide was molten and stirred at 55°C and in total 100g of acetone was added slowly taking care the temperature remained above 52°C. This solution was subsequently allowed to statically cool to room temperature overnight under ambient conditions.

The following table provides feed and product properties.

| | Free acid (meq/kg) | Meso-lactide (wt.%) |
|---|---|---|
| Feed | 185 | 91 |
| Product crystals - before washing | 43 | 99 |
| Product crystals - after washing with acetone | 22 | 99 |

It can be seen that static solvent crystallisation is also capable of yielding meso-lactide crystals with a decreased free acid content. It furthermore provides evidence that an optional washing step may yield further purification, if so desired. Care should be taken in such further washing step to limit the dissolution of the crystals a far as possible as this will affect the yield.

### Example 8 (comparative): higher solvent content

In this example the effect of using more than 50 wt.% of solvent was investigated. 500g meso-lactide was molten and stirred at 55°C and in total 750 grams of acetone was added slowly taking care the temperature remained above 52°C. The solvent content was 60 wt.%, calculated on the total of meso-lactide and solvent. This solution was cooled, and meso-lactide seeds were added at various temperatures. The results are given in the table.

| Temperature [°C] | Seed addition |
|---|---|
| 10 | Seeds dissolve |
| 5 | Seeds dissolve |
| 0 | Seeds dissolve |
| -5 | Seeds dissolve |

It appeared that at each test temperature, the seeds dissolved. This means that for this amount of solvent crystallisation at an economically attractive temperature is not possible.

### Example 9 (comparative): isopropanol as solvent

Analogous to what is described in Example 1, solid meso-lactide particles were combined with isopropanol as solvent, in an amount of 20 wt.% (calculated on the total of meso-lactide and isopropanol). The mixture of solvent and meso-lactide was heated in a double-jacketed, temperature controlled vessel with bottom glass filter, with the aim to dissolve the meso-lactide to carry out solvent crystallisation. It appeared however, that the solubility of the meso-lactide in isopropanol was so low that that it was not possible to dissolve the meso-lactide in the isopropanol at a temperature below the melting point of the meso-lactide. Accordingly, it was not possible to carry out solvent crystallisation in the presence of 20 wt.% of isopropanol. Further investigation of the solubility of meso-lactide in isopropanol showed that the ability of isopropanol to solubilize meso-lactide is significantly lower than in the case of the solvents according to the invention. It should also be noted that the presence of residual alcohol solvent limits the maximum attainable molecular weight of polymer manufactured from the lactide, because the alcohol may react in the esterification reaction.

## Claims

1. Process for purifying meso-lactide which has a free acid content of at least 30 meq/kg comprising at least one step of
subjecting a feed comprising at least 75 wt.% meso-lactide to solvent crystallisation followed by recovery of product meso-lactide, wherein the solvent crystallisation step is carried in a solvent comprising a total of at least 80 wt.% of at least one compound selected from the group of ketones of the formula R1-C(=O)-R2, wherein R1 and R2 are independently selected from methyl, ethyl, propyl, isopropyl, n-butyl, sec-butyl, t-butyl, and isobutyl and ethers of the formula R3-O-R4, wherein R3 and R4 are independently selected from methyl, ethyl, propyl, isopropyl, n-butyl, sec-butyl, t-butyl, and isobutyl, the solvent being present in an amount of 5-50 wt.% calculated on the total of feed and solvent, the solvent crystallisation conditions being selected such that at least 20% of the meso-lactide in the feed crystallises in solid form,
wherein the solid meso lactide recovered from a final step of solvent crystallisation followed by recovery of product meso-lactide has a free acid content of at most 20 meq/kg,
in which a total of 2-10 sequential steps of solvent crystallisation followed by recovery of product meso-lactide are carried out, and wherein at least one of the solvent crystallisation steps is carried out under the reduction of the temperature of the mixture of solvent and feed to a value below 50°C.

2. Process according to claim 1, in which a total of 2-6 sequential steps of solvent crystallisation followed by recovery of product meso-lactide are carried out.

3. Process according to any one of the preceding claims, wherein the solvent crystallisation step is carried in a solvent consisting for at least 90 wt.%, more in particular at least 95 wt.%, even more in particular at least 98 wt.% of the total of at least one compound selected from the group of ketones and ethers as specified in claim 1, in particular of a compound selected from the group of ketones and ethers as specified in claim 1.

4. Process according to any one of the preceding claims, wherein in at least one, and preferably all, of the steps of solvent crystallisation followed by recovery of product meso-lactide a ketone solvent is used in which R1 is methyl, with R2 preferably being selected from methyl, ethyl, and isobutyl, wherein R1 and R2 are more preferably both methyl.

5. Process according to any one of claims 1-3, wherein in at least one, and preferably all, of the steps of solvent crystallisation followed by recovery of product meso-lactide an ether solvent is used in which R3 and R4 are independently selected from methyl, ethyl, or isopropyl, wherein R3 and R4 are more preferably both isopropyl, in particular wherein acetone is used as solvent.

6. Process according to any one of the preceding claims, wherein at least one, preferably all of the solvent crystallisation steps are carried out such that at least 25% of the meso-lactide in the feed crystallises in solid form, in particular at least 30%, and/or at most 80%.

7. Process according to any one of the preceding claims, wherein the feed comprising meso-lactide has a free acid content of at least 50 meq/kg, preferably at least 80 meq/kg, in particular at least 120 meq/kg, or at least 150 meq/kg, and/or at most 700 meq/kg in particular at most 400 meq/kg, more in particular at most 300 meq/kg, often at most 200 meq/kg.

8. Process according to any one of the preceding claims, wherein at most 40 wt.% solvent is used, in particular at most 35 wt.%, more in particular at most 30 wt.%, and in particular at least 10 wt.%.

9. Process according to any one of the preceding claims, wherein the solid meso lactide recovered from the final step of solvent crystallisation followed by recovery of product meso-lactide has a free acid content of at most 15 meq/kg, in particular at most 10 meq/kg, e.g., at most 5 meq/kg, at most 2 meq/kg, or at most 1 meq/kg.

10. Process according to any one of the preceding claims, wherein at least one, preferably all, of the solvent crystallisation steps are carried out under the reduction of the temperature of the mixture of solvent and feed to a value below 40°C, in particular below 30°C and/or at least 10°C.

11. Process for producing purified meso-lactide and a purified L-lactide and/or D-lactide stream comprising the steps of
a) reacting lactic acid to form low molecular weight poly(lactic acid),
b) depolymerising the low molecular weight poly(lactic acid) to form a crude lactide comprising meso-lactide, and L-lactide and/or D-lactide wherein either L-lactide or D-lactide is the non-predominant lactide,
c) separating meso-lactide from the crude lactide in one or more steps, resulting in the formation of a meso-lactide stream comprising at least 75 wt.% of meso-lactide which has a free acid content of at least 30 meq/kg and a purified L-lactide and/or D-lactide stream,
d) and subjecting the meso-lactide stream comprising at least 75 wt.% of meso-lactide which has a free acid content of at least 30 meq/kg to a purification process, wherein the purification process is a process in accordance with any one of claims 1-10, and
recovering a purified meso-lactide and a purified L-lactide and/or D-lactide stream.

12. Process according to any one of the preceding claims, wherein the purified meso-lactide and/or a purified L-lactide and/or D-lactide stream is converted to high purity lactic acid by hydrolysis with water.

13. Process for producing polylactide comprising the steps of
a) reacting lactic acid to form low molecular weight poly(lactic acid),
b) depolymerising the low molecular weight poly(lactic acid) to form a crude lactide comprising meso-lactide, and L-lactide and/or D-lactide wherein either L-lactide or D-lactide is the non-predominant lactide,
c) separating meso-lactide from the crude lactide in one or more steps, resulting in the formation of a meso-lactide stream comprising at least 75 wt.% of meso-lactide which has a free acid content of at least 30 meq/kg and a purified L-lactide and/or D-lactide stream,
d) subjecting the meso-lactide stream comprising at least 75 wt.% of meso-lactide which has a free acid content of at least 30 meq/kg to a purification process, wherein the purification process is a process according to any one of claims 1-10, and
e) subjecting at least a portion of the purified L-lactide and/or D-lactide stream, at least a portion of the purified meso-lactide, or the combination of at least a portion of the purified L-lactide and/or D-lactide stream and at least a portion of the purified meso-lactide to a polymerisation step to form polylactide.

14. Process according to claim 13, wherein the combination of at least a portion of the purified L-lactide and/or D-lactide stream and at least a portion of the purified meso-lactide is provided to a polymerisation step to form polylactide.

15. Process according to claim 13 or 14, wherein at least a portion of the purified meso-lactide formed in step d) is provided to step a) or step b).

## Patentansprüche

1. Verfahren zur Reinigung von meso-Lactid, das einen Gehalt an freier Säure von mindestens 30 mÄq/kg aufweist, umfassend mindestens einen Schritt des Unterziehens einer Beschickung, die mindestens 75 Gew.-% meso-Lactid umfasst, einer Lösungsmittelkristallisation, gefolgt von Gewinnung des meso-Lactid-Produkts, wobei der Lösungsmittelkristallisationsschritt in einem Lösungsmittel durchgeführt wird, das insgesamt mindestens 80 Gew.-% mindestens einer Verbindung, ausgewählt aus der Gruppe von Ketonen der Formel R1-C(=O)-R2 , wobei R1 und R2 unabhängig aus Methyl, Ethyl, Propyl, Isopropyl, n-Butyl, sec-Butyl, t-Butyl und Isobutyl ausgewählt sind, und Ethern der Formel R3-O-R4, wobei R3 und R4 unabhängig aus Methyl, Ethyl, Propyl, Isopropyl, n-Butyl, sec-Butyl, t-Butyl und Isobutyl ausgewählt sind, umfasst, wobei das Lösungsmittel in einer Menge von 5 bis 50 Gew.-%, berechnet bezogen auf die Gesamtmenge aus Beschickung und Lösungsmittel, vorliegt, wobei die Lösungsmittelkristallisationsbedingungen derart gewählt sind, dass mindestens 20% des meso-Lactids in der Beschickung in fester Form kristallisieren,
wobei das feste meso-Lactid, das aus einem letzten Lösungsmittelkristallisationschritt, gefolgt von Gewinnung des meso-Lactid-Produkts, gewonnen wird, einen Gehalt an freier Säure von höchstens 20 mÄq/kg aufweist, wobei insgesamt 2 bis 10 aufeinanderfolgende Lösungsmittelkristallisationsschritte, gefolgt von Gewinnung des meso-Lactid-Produkts, durchgeführt werden und wobei mindestens einer der Lösungsmittelkristallisationsschritte unter der Verringerung der Temperatur des Gemischs aus Lösungsmittel und Beschickung auf einen Wert unter 50°C durchgeführt wird.

2. Verfahren nach Anspruch 1, wobei insgesamt 2 bis 6 aufeinanderfolgende Schritte der Lösungsmittelkristallisation, gefolgt von Gewinnung des meso-Lactid-Produkts, durchgeführt werden.

3. Verfahren nach einem der vorhergehenden Ansprüche, wobei der Lösungsmittelkristallisationsschritt in einem Lösungsmittel durchgeführt wird, das zu mindestens 90 Gew.-%, insbesondere mindestens 95 Gew.-%, noch mehr insbesondere mindestens 98 Gew.-% der Gesamtmenge mindestens einer Verbindung, ausgewählt aus der Gruppe von Ketonen und Ethern nach Anspruch 1, insbesondere einer Verbindung, ausgewählt aus der Gruppe von Ketonen und Ethern nach Anspruch 1, besteht.

4. Verfahren nach einem der vorhergehenden Ansprüche, wobei in mindestens einem, und vorzugsweise allen Lösungsmittelkristallisationsschritten, gefolgt von Gewinnung des meso-Lactid-Produkts, ein Ketonlösungsmittel verwendet wird, in dem R1 Methyl ist, wobei R2 vorzugsweise aus Methyl, Ethyl und Isobutyl ausgewählt ist, wobei R1 und R2 noch mehr bevorzugt beide Methyl sind.

5. Verfahren nach einem der Ansprüche 1-3, wobei in mindestens einem, und vorzugsweise allen Lösungsmittelkristallisationsschritten, gefolgt von Gewinnung des meso-Lactid-Produkts, ein Etherlösungsmittel verwendet wird, in dem R3 und R4 unabhängig aus Methyl, Ethyl oder Isopropyl ausgewählt sind, wobei R3 und R4 noch mehr bevorzugt beide Isopropyl sind, insbesondere wobei Aceton als Lösungsmittel verwendet wird.

6. Verfahren nach einem der vorhergehenden Ansprüche, wobei mindestens einer, vorzugsweise alle Lösungsmittelkristallisationsschritte derart durchgeführt werden, dass mindestens 25%, insbesondere mindestens 30% und/oder höchstens 80% des meso-Lactids in der Beschickung in fester Form kristallisieren.

7. Verfahren nach einem der vorhergehenden Ansprüche, wobei die meso-Lactid umfassende Beschickung einen Gehalt an freier Säure von mindestens 50 mÄq/kg, vorzugsweise mindestens 80 mÄq/kg, insbesondere mindestens 120 mÄq/kg oder mindestens 150 mÄq/kg und/oder höchstens 700 mÄq/kg, insbesondere höchstens 400 mÄq/kg, noch mehr insbesondere höchstens 300 mÄq/kg, häufig höchstens 200 mÄq/kg aufweist.

8. Verfahren nach einem der vorhergehenden Ansprüche, wobei höchstens 40 Gew.-% Lösungsmittel, insbesondere höchstens 35 Gew.-%, noch mehr insbesondere höchstens 30 Gew.-% und insbesondere mindestens 10 Gew.-% verwendet werden.

9. Verfahren nach einem der vorhergehenden Ansprüche, wobei das feste meso-Lactid, das aus dem letzten Lösungsmittelkristallisationsschritt, gefolgt von Gewinnung des meso-Lactid-Produkts, gewonnen wird, einen Gehalt an freier Säure von höchstens 15 mÄq/kg, insbesondere höchstens 10 mÄq/kg, z.B. höchstens 5 mÄq/kg, höchstens 2 mÄq/kg oder höchstens 1 mÄq/kg aufweist.

10. Verfahren nach einem der vorhergehenden Ansprüche, wobei mindestens einer, vorzugsweise alle Lösungsmittelkristallisationsschritte unter Verringerung der Temperatur des Gemischs aus Lösungsmittel und Beschickung auf einen Wert unter 40°C, insbesondere unter 30°C und/oder mindestens 10°C durchgeführt werden.

11. Verfahren zur Herstellung von gereinigtem meso-Lactid und eines gereinigten L-Lactid- und/oder D-Lactid-Stroms, umfassend die Schritte
a) Umsetzen von Milchsäure unter Bildung von Poly(milchsäure) mit niedrigem Molekulargewicht,
b) Depolymerisieren der Poly(milchsäure) mit niedrigem Molekulargewicht unter Bildung eines rohen Lactids, das meso-Lactid und L-Lactid und/oder D-Lactid umfasst, wobei entweder L-Lactid oder D-Lactid das nicht vorherrschende Lactid ist,
c) Abtrennen von meso-Lactid aus dem rohen Lactid in einem oder mehreren Schritten, was zur Bildung eines meso-Lactid-Stroms, der mindestens 75 Gew.-% meso-Lactid umfasst, das einen Gehalt an freier Säure von mindestens 30 mÄq/kg aufweist, und eines gereinigten L-Lactid- und/oder D-Lactid-Stroms führt,
d) und Unterziehen des meso-Lactid-Stroms, der mindestens 75 Gew.-% meso-Lactid umfasst, das einen Gehalt an freier Säure von mindestens 30 mÄq/kg aufweist, einem Reinigungsverfahren, wobei das Reinigungsverfahren ein Verfahren nach einem der Ansprüche 1-10 ist, und Gewinnen eines gereinigten meso-Lactids und eines gereinigten L-Lactid- und/oder D-Lactidstroms.

12. Verfahren nach Anspruch 11, wobei das gereinigte meso-Lactid und/oder ein gereinigter L-Lactid- und/oder D-Lactid-Strom durch Hydrolyse mit Wasser in hochreine Milchsäure umgewandelt wird.

13. Verfahren zur Herstellung von Polylactid, umfassend die Schritte
a) Umsetzen von Milchsäure unter Bildung von Poly(milchsäure) mit niedrigem Molekulargewicht,
b) Depolymerisieren der Poly(milchsäure) mit niedrigem Molekulargewicht unter Bildung eines rohen Lactids, das meso-Lactid und L-Lactid und/oder D-Lactid umfasst, wobei entweder L-Lactid oder D-Lactid das nicht vorherrschende Lactid ist,
c) Abtrennen von meso-Lactid aus dem rohen Lactid in einem oder mehreren Schritten, was zur Bildung eines meso-Lactid-Stroms, der mindestens 75 Gew.-% meso-Lactid umfasst, das einen Gehalt an freier Säure von mindestens 30 mÄq/kg aufweist, und eines gereinigten L-Lactid- und/oder D-Lactid-Stroms führt,
d) Unterziehen des meso-Lactid-Stroms, der mindestens 75 Gew.-% meso-Lactid umfasst, das einen Gehalt an freier Säure von mindestens 30 mÄq/kg aufweist, einem Reinigungsverfahren, wobei das Reinigungsverfahren ein Verfahren nach einem der Ansprüche 1 - 10 ist, und
e) Unterziehen mindestens eines Teils des gereinigten L-Lactid- und/oder D-Lactid-Stroms, mindestens eines Teils des gereinigten meso-Lactids oder der Kombination von mindestens einem Teil des gereinigten L-Lactid- und/oder D-Lactid-Stroms und mindestens einem Teil des gereinigten meso-Lactids einem Polymerisationsschritt, um Polylactid zu bilden.

14. Verfahren nach Anspruch 13, wobei die Kombination von mindestens einem Teil des gereinigten L-Lactid- und/oder D-Lactid-Stroms und mindestens einem Teil des gereinigten meso-Lactids einem Polymerisationsschritt zugeführt wird, um Polylactid zu bilden.

15. Verfahren nach Anspruch 13 oder 14, wobei mindestens ein Teil des in Schritt d) gebildeten gereinigten meso-Lactids Schritt a) oder Schritt b) zugeführt wird.

## Revendications

1. Processus de purification de méso-lactide qui présente une teneur en acide libre d'au moins 30 meq/kg comprenant au moins une étape parmi
la soumission d'une charge comprenant au moins 75 % en poids de méso-lactide à une cristallisation par solvant, suivie de la récupération du méso-lactide résultant, dans lequel l'étape de cristallisation par solvant est réalisée dans un solvant comprenant un total d'au moins 80 % en poids d'au moins un composé choisi dans le groupe des cétones de la formule R1-C(=O)-R2, dans lequel R1 et R2 sont choisis indépendamment parmi méthyle, éthyle, propyle, isopropyle, n-butyle, sec-butyle, t-butyle et isobutyle, ainsi que des éthers de la formule R3-O-R4, dans lequel R3 et R4 sont choisis indépendamment parmi méthyle, éthyle, propyle, isopropyle, n-butyle, sec-butyle, t-butyle et isobutyle, le solvant étant présent en une quantité de 5-50 % en poids, calculée sur le total de la charge et du solvant, les conditions de cristallisation par solvant étant choisies de sorte qu'au moins 20 % du méso-lactide dans la charge cristallise sous forme solide,
dans lequel le méso-lactide solide, récupéré à partir d'une étape finale de cristallisation par solvant suivie de la récupération du méso-lactide résultant, présente une teneur en acide libre d'au plus 20 meq/kg, dans lequel un total de 2-10 étapes séquentielles de cristallisation par solvant, suivies de la récupération du méso-lactide résultant sont réalisées, et dans lequel au moins l'une des étapes de cristallisation par solvant est effectuée en abaissant la température du mélange de solvant et de charge à une valeur inférieure à 50 °C.

2. Processus selon la revendication 1, dans lequel un total de 2-6 étapes séquentielles de cristallisation par solvant suivies d'une récupération de méso-lactide résultant sont réalisées.

3. Processus selon quelconque des revendications précédentes, dans lequel l'étape de cristallisation par solvant est réalisée dans un solvant constituant au moins 90 % en poids, plus particulièrement au moins 95 % en poids, et encore plus particulièrement au moins 98 % en poids du total d'au moins un composé choisi dans le groupe des cétones et des éthers tel que spécifié dans la revendication 1, en particulier d'un composé choisi dans le groupe des cétones et des éthers tel que spécifié dans la revendication 1.

4. Processus selon quelconque des revendications précédentes, dans lequel dans au moins une, et de préférence la totalité, des étapes de cristallisation par solvant suivies de la récupération du méso-lactide résultant, un solvant cétone est utilisé, dans lequel R1 est méthyle, R2 étant de préférence choisi parmi méthyle, éthyle et isobutyle, dans lequel R1 et R2 sont de manière davantage préférée tous deux méthyle.

5. Processus selon quelconque des revendications 1-3, dans lequel dans au moins une, et de préférence la totalité, des étapes de cristallisation suivies de la récupération du méso-lactide résultant, un solvant éther est utilisé dans lequel R3 et R4 sont choisis indépendamment parmi méthyle, éthyle ou isopropyle, dans lequel R3 et R4 sont de préférence tous deux isopropyle, en particulier dans lequel l'acétone est utilisée comme solvant.

6. Processus selon quelconque des revendications précédentes, dans lequel au moins une, de préférence la totalité, des étapes de cristallisation par solvant sont réalisées de sorte qu'au moins 25 % du méso-lactide dans la charge cristallise sous forme solide, en particulier au moins 30 %, et/ou au plus 80 %.

7. Processus selon quelconque des revendications précédentes, dans lequel la charge comprenant du méso-lactide présente une teneur en acide libre d'au moins 50 meq/kg, de préférence d'au moins 80 meq/kg, en particulier d'au moins 120 meq/kg, ou d'au moins 150 meq/kg, et/ou d'au plus 700 meq/kg, en particulier d'au plus 400 meq/kg, plus particulièrement d'au plus 300 meq/kg, et souvent d'au plus 200 meq/kg.

8. Processus selon quelconque des revendications précédentes, dans lequel au plus 40 % en poids de solvant sont utilisés, en particulier au plus 35 % en poids, plus particulièrement au plus 30 % en poids, et en particulier au moins 10 % en poids.

9. Processus selon quelconque des revendications précédentes, dans lequel le méso-lactide solide récupéré lors de l'étape finale de cristallisation par solvant, suivie de la récupération du méso-lactide résultant, présente une teneur en acide libre d'au plus 15 meq/kg, en particulier d'au plus 10 meq/kg, par exemple d'au plus 5 meq/kg, d'au plus 2 meq/kg, ou d'au plus 1 meq/kg.

10. Processus selon quelconque des revendications précédentes, dans lequel au moins une, de préférence la totalité, des étapes de cristallisation par solvant sont réalisées en abaissant la température du mélange de solvant et de charge à une valeur inférieure à 40 °C, en particulier inférieure à 30 °C et/ou à au moins 10 °C.

11. Processus de production de méso-lactide purifié et d'un flux de L-lactide et/ou de D-lactide purifié comprenant les étapes suivantes
a) la réaction de l'acide lactique pour former du poly(acide lactique) de faible poids moléculaire,
b) la dépolymérisation du poly(acide lactique) de faible poids moléculaire pour former un lactide brut comprenant du méso-lactide, et du L-lactide et/ou du D-lactide dans lequel le L-lactide ou le D-lactide est le lactide non prédominant,
c) la séparation du méso-lactide du lactide brut en une ou plusieurs étapes, entraînant la formation d'un flux de méso-lactide comprenant au moins 75 % en poids de méso-lactide qui présente une teneur en acide libre d'au moins 30 meq/kg, et d'un flux de L-lactide et/ou de D-lactide purifié,
d) et la soumission du flux de méso-lactide comprenant au moins 75 % en poids de méso-lactide qui présente une teneur en acide libre d'au moins 30 meq/kg à un processus de purification, dans lequel le processus de purification est un processus conforme à quelconque des revendications 1-10, et la récupération d'un flux de méso-lactide purifié et d'un flux de L-lactide et/ou de D-lactide purifié.

12. Processus selon quelconque des revendications précédentes, dans lequel le méso-lactide purifié et/ou un flux de L-lactide et/ou de D-lactide purifié est converti en acide lactique de haute pureté par hydrolyse avec de l'eau.

13. Processus de production de polylactide comprenant les étapes suivantes
a) la réaction de l'acide lactique pour former du poly(acide lactique) de faible poids moléculaire,
b) la dépolymérisation du poly(acide lactique) de faible poids moléculaire pour former un lactide brut comprenant du méso-lactide, et du L-lactide et/ou du D-lactide dans lequel le L-lactide ou le D-lactide est le lactide non prédominant,
c) la séparation du méso-lactide du lactide brut en une ou plusieurs étapes, entraînant la formation d'un flux de méso-lactide comprenant au moins 75 % en poids de méso-lactide qui présente une teneur en acide libre d'au moins 30 meq/kg, et d'un flux de L-lactide et/ou de D-lactide purifié,
d) la soumission du flux de méso-lactide comprenant au moins 75 % en poids de méso-lactide qui présente une teneur en acide libre d'au moins 30 meq/kg à un processus de purification, dans lequel le processus de purification est un processus conforme à quelconque des revendications 1-10, et
e) la soumission d'au moins une partie du flux de L-lactide et/ou de D-lactide purifié, d'au moins une partie du méso-lactide purifié, ou de la combinaison d'au moins une partie du flux de L-lactide et/ou de D-lactide purifié et d'au moins une partie du méso-lactide purifié à une étape de polymérisation pour former du polylactide.

14. Processus selon la revendication 13, dans lequel la combinaison d'au moins une partie du flux de L-lactide et/ou de D-lactide purifié et d'au moins une partie du méso-lactide purifié est soumise à une étape de polymérisation pour former du polylactide.

15. Processus selon la revendication 13 ou 14, dans lequel au moins une partie du méso-lactide purifié formé à l'étape d) est fournie à l'étape a) ou à l'étape b).
